# EUROPEAN PATENT SPECIFICATION

(11) **EP 4 160 476 B1**
(45) Date of publication and mention of the grant of the patent: **02.12.2026**
(21) Application number: 21200561.5
(22) Date of filing: 01.10.2021
(51) Int. Cl.: C12Q 1/10, G06F 18/2413, G06V 20/69

(54) **A METHOD AND SYSTEM FOR ANALYZING INTESTINAL MICROFLORA OF A SUBJECT**
VERFAHREN UND SYSTEM ZUR ANALYSE DER DARMMIKROFLORA EINES PATIENTEN
PROCÉDÉ ET SYSTÈME D'ANALYSE DE LA MICROFLORE INTESTINALE D'UN SUJET

(43) Date of publication of application: 05.04.2023
(73) Proprietor: HORAIZON technology B.V., 2645 LT Delfgauw (NL)
(72) Inventor: MAASLAND, Troy, 2645 LT Delfgauw (NL); LEVIN, Evgeni, 2645 LT Delfgauw (NL)
(74) Representative: V.O.

(56) References cited:
- LIU LIN ET AL: "Automatic identification of fungi under complex microscopic fecal images", JOURNAL OF BIOMEDICAL OPTICS, vol. 20, no. 7, 13 July 2015 (2015-07-13), 1000 20th St. Bellingham WA 98225-6705 USA, pages 076004, XP055899341, ISSN: 1083-3668, DOI: 10.1117/1.JBO.20.7.076004
- WAHID MD FERDOUS ET AL: "Classification of Microscopic Images of Bacteria Using Deep Convolutional Neural Network", 2018 10TH INTERNATIONAL CONFERENCE ON ELECTRICAL AND COMPUTER ENGINEERING (ICECE), IEEE, 20 December 2018 (2018-12-20), pages 217 - 220, XP033514924, [retrieved on 20190206], DOI: 10.1109/ICECE.2018.8636750

## Description

### FIELD OF THE DISCLOSURE

The disclosure relates to a method analyzing intestinal microflora of a subject (e.g. estimating intestinal microflora composition). The disclosure also relates to a system configured for analyzing intestinal microflora of a subject. Furthermore, the disclosure relates to a method of determining an indication of a subject's health. Additionally, the disclosure relates to a method of training a machine learning model for analyzing intestinal microflora of a subject. Also, the disclosure relates to a computer program product.

### BACKGROUND TO THE DISCLOSURE

It is well known that the gut microbiome can provide valuable insights into the health and lifestyle habits of a subject. Specific lifestyle habits such as a diet may have an impact on the intestinal microflora of the subject.

Determining a relationship between the subject and its bacterial colonizers (cf. host-microbiome relationship) may provide valuable insights, which can benefit for example digestion, the immune system and various other aspects of health. Such relationship which exists in a symbiotic state may be exposed to environmental and host-specific factors which may cause variations. For example, some events or dietary habits of the host may alter the relationship. In extreme cases, for example as a result of extreme stress, alcohol misuse, medication, inappropriate diet, disease, allergies, sleep deprivation, etc., the imbalance in the microflora may cause dysbiosis or breakdown of the relationship and interactions. This may result in important changes in the functional composition and metabolic activities.

An imbalance of unhealthy and healthy microbes in the intestines may contribute to health issues, such as weight gain, high blood sugar, high cholesterol, etc. Nevertheless, the gut microbiome is very adaptable and typically responds to positive lifestyle choices. The diet of the subject may be an important modifiable factor influencing the composition of the gut microbiota. Hence, therapeutic dietary strategies may be employed in order to manipulate microbial diversity, composition, and stability. Other ways to influence microbial composition as well as health status of the individuals includes Fecal Microbial Transplants (FMT) which have been used prove causal role of microbiome for variety of health conditions. For instance, a fiber-rich diet, intake of probiotics or particular supplements may speed up the gut biome restoration. The gut microbiota of the subject may play a key role in personalized nutrition.

Unfortunately, it may be cumbersome to adequately analyze the intestinal microflora. Although rapid progress in DNA sequencing technology has provided ever-increasing outputs coupled with lowered costs, facilitating an explosion in amplicon sequencing studies, it remains a barrier in many cases. In this way, the current technologies are not suited for efficient monitoring of the microbiota of subjects.

There is a strong need for a faster and more efficient process for effectively analyzing and estimating intestinal microflora of a subject.

The paper "Automatic identification of fungi under complex microscopic fecal images", Lin Liu et al., Journal of Biomedical Optics 20(7), 076004 (July 2015), discloses that automatic identification of fungi in microscopic fecal images provides important information for evaluating digestive diseases. The proposed system automatically identifies fungi in microscopic fecal images that contain other cells and impurities under complex environments. Images are segmented twice to obtain the correct area of interest, and ten features are selected, including the circle number, concavity point, and other basic features, to filter fungi. An artificial neural network (ANN) system is used to identify the fungi. The first stage (ANN-1) processes features from five images in differing focal lengths; the second stage (ANN-2) identifies the fungi using the ANN-1 output values. Images in differing focal lengths can be used to improve the identification result. The system output detects the image, whether or not it has fungi. If the image does have fungi, the system output counts the number of different fungi.

The paper "Classification of Microscopic Images of Bacteria Using Deep Convolutional Neural Network", Md. Ferdous Wahid et al., 10th International Conference on Electrical and Computer Engineering, 20-22 December, 2018, Dhaka, Bangladesh, discloses that classification of bacteria is essential in medical science for diagnosis of numerous diseases, treatment of infection, and trace-back of disease outbreaks. The paper investigates an approach to automate the process of bacteria recognition and classification with the use of deep convolutional neural network (DCNN). It used the 'transfer learning' method to retrain the 'Inception DCNN model' with of a dataset of more than 500 microscopic images of 5 different bacteria species that are harmful to human-health. 20% images of the dataset were randomly chosen and separated, which were used to test the classification accuracy of the network. The retrained model was able to recognize and classify all 5 different species of bacteria, while the experimental results of prediction achieved accuracy of around 95%.

### SUMMARY

The invention is defined by the set of appended claims. It is an object of the disclosure to provide for a method and a system that obviates at least one of the above-mentioned drawbacks.

Additionally or alternatively, it is an object of the disclosure to improve the analysis and/or characterization of intestinal microflora of a subject.

Additionally or alternatively, it is an object of the disclosure to improve the efficiency of microbiome analysis.

Additionally or alternatively, it is an object of the disclosure to provide for a cost-effective method and system for analyzing (e.g. estimating) the intestinal microbiome of a subject.

Thereto, the disclosure provides for a method for analyzing (e.g. estimating) intestinal microflora of a subject according to claim 1.

Images of fecal samples are used for rapid and efficient analysis of the microbiome. The analysis can for example be used for rapid microbiome profiling and/or enterotype estimation, only using an image of the fecal sample or features extracted from said image. Advantageously, laboratory analysis is not needed. The process becomes less cumbersome and allows more easy, efficient and cost-effective monitoring of the microbiome of the subject. Additionally, this allows to perform multiple tests during a period of time, which would be unpractical if laboratory tests were to be conducted. For example, an image of a fecal sample may be monitored every day in order to track changes in the microbiome of the subject. As a result, it becomes significantly more easy to provide for personalized diet. It can be determined whether the diet of the subject has to be adjusted in order to improve the microbial balance (cf. prevent microbial imbalance). Thus, advantageously, impaired microbiota can be better prevented in this way.

The digital image is an image of a fecal streak on a substrate.

Utilizing a fecal streak on a substrate can significantly improve the performance of the trained machine learning model. Furthermore, the machine learning model can be trained using less data. The training process can thus become more efficient if training data with fecal streaks are used for training the machine learning model (e.g. deep learning network model). This is an important benefit, as obtaining the training data may require expensive (laboratory) tests, for instance involving DNA/RNA sequencing.

Furthermore, as the accuracy of the trained machine learning model can be enhanced by using an image of a fecal streak on a substrate, it is possible to better prevent impaired microbiota of the subject. The trained machine learning model can provide improved predictability, whilst an efficient and simple method can be used. It is rather easy to make an image of a fecal streak on a substrate. In some cases, this image may be provided by a non-professional, for instance the subject may take the image of the fecal streak on a substrate.

The fecal streak is obtained by smearing a layer of feces on the substrate.

By smearing a sample of feces on the substrate, a relatively thin layer of feces can be obtained. Such a thin layer of feces, which allow trained machine learning model to extract various features of the image such as but not limited to composition, color, texture, granularity and many others, may increase the performance of the trained machine learning model, and also improve training of the machine learning model.

Optionally, the thickness of the fecal streak layer is smaller than 3000 micrometer, more preferably smaller than 1000 micrometer, even more preferably smaller than 500 micrometer.

Optionally, the layer of feces is smeared on the substrate by means of a blade (or a brush). Using a blade, a relatively thin layer of feces can be applied on the substrate rather easily. In this way, a more uniform distribution of the feces over the substrate can be obtained. In this way, the grittiness of the feces may be more easily captured in the image. For instance, the granularity or structure of the feces sample can be better captured, resulting in improved performance and training of the machine learning model.

Optionally, the substrate is a sheet of paper, such as white paper, e.g. having a TAPPI brightness of 92 or higher.

A sheet of paper is readily available and can provide easily sufficient contrast in the image of the sample of feces of the subject, under normal lighting condition. Additionally, by using a sheet of paper, a cost-effective solution can be obtained.

Optionally, the substrate and/or an area covered by the fecal streak have dimensions falling within a predetermined range.

In some examples, a sheet of paper with a predetermined size is used, for instance a sheet of paper in A4 format (or any other format). It is also possible that some region of a substrate is used for smearing the sample of feces. In some examples, a substrate is provided with a pre-marked region in which the feces sample has to be smeared. For example, a rectangle, circle, square, etc. region may be marked on a sheet of A4 paper. Optionally, the substrate may be printable by means of a printer based on an electronic format file, such as a Portable Document Format (PDF) file. This allows users for easily obtaining the substrate. Such electronic file may be easily provided to the user via a mobile app, such as an Android or iOS app or the like.

The image classification is associated to abundances of predetermined microbial species, genera and other phylogenetic information.

Visual properties of the fecal samples, which can be identified by image detection models such as a trained machine learning model, can be linked to a subject's microbiome. The composition of the gut microbiota in subjects may be associated to the health condition of the subject. The dietary composition can be a significant target in a strategy aiming at monitoring the health of the subject, or even prevention of metabolic diseases. The health monitoring can be made more easy by utilizing images of fecal streaks on a substrate. In this way, the scale of health monitoring can be improved, since a large number of subjects may provide their own images, for instance via a mobile application. The analysis can be performed on-the-fly using a mobile app. The images of the users may be uploaded to a server which is configured to analyze the images using the trained machine learning model. The classification output of the trained machine learning model may optionally be further post-processed and provided back to the user and/or to one or more health professionals.

Advantageously, personalized nutrition may become available to a wider range of people. Recent studies have observed a high variability in the effects on glucose tolerance to food between subjects although the meals or more specific food items were identical. For example, based on the analysis of the image of the fecal sample, a glucose response can be predicted by collecting data regarding personal features and/or gut microbiota composition. Hence, a personalized diet may be easily built taking into account the analysis of the intestinal microflora of the subject.

Tthe predetermined microbial genera include one or more microbial enterotypes or microbial abundance profiles, including prevotella and/or bacteroides.

A diverse array of microbes may colonize the human intestine. The analysis of the intestinal microbiota may be used for characterizing a healthy or normal intestinal microbiome. It can also provide insights in what factors modify the intestinal microbiome in the healthy state and how the intestinal microbiome affects normal host physiology. Such factors may be different for different subjects, thus allowing a personalized assessment. A number of factors, including age, host immune system, host genetics, diet and medication use (e.g. antibiotic use) appear to modify the intestinal microbiome in the normal state to some extent. The microbiome may play a critical role in the healthy human immune system and metabolism.

The method includes determining, based on the output image classification, relative abundances of the one or more predetermined microbial enterotypes, species or genera, including a ratio of the abundance of prevotella genera and the abundance of bacteroides genera.

A bi-directional relationship between the intestinal microbiota and the subject can be vital to health. The disclosure enables easy and efficient monitoring data indicative of the composition of the intestinal microbiota An enhanced understanding of such relationship can be used for improving the health of the subject. Additionally or alternatively, it can be employed for better understanding disease, which can result in improved therapeutic approaches.

Optionally, the machine learning model has been trained using a data set comprising a plurality of digital images of fecal samples, each digital image accompanied by microbial measurement data representative of abundances of the predetermined microbial enterotype, species or genera in the fecal sample in the digital image.

Optionally, the measurement data includes sequencing data, such as but not limited to 16S ribosomal RNA or shotgun sequencing data, corresponding to the fecal sample in the digital image.

Optionally, the measurement data accompanying each digital image is statistically normalized to a predetermined total abundance of the predetermined microbial genera.

Optionally, the data set used for training is augmented using one or more image transformations, such as cropping, rotation, translation, and color change.

According to an aspect, the disclosure provides for a method of determining an indication of a subject's health based on data indicative of one or more properties of the intestinal microflora of the subject according to claim 4.

Advantageously, the method can be used for performing dietary interventions. For instance, saturated fats and animal protein, which are typically high in a Western diet, are associated with metabolic disorders such as obesity, reduced insulin sensitivity, type 2 diabetes, and cardiovascular disease, and also altered microbiota, e.g., reduced diversity. For example, dietary fiber can have preventive potential on cardiometabolic risk, and may be employed in a personalized diet.

Optionally, the digital image of the sample of feces of the subject is taken by means of a mobile device and uploaded to a server, wherein the server is configured to carry out the method according to the disclosure.

In this way, a user friendly method can be obtained, reducing the barrier for (frequent) monitoring of the intestinal microflora. The users themselves may provide the images, for instance by uploading the images via a mobile app. In some cases, this would not require any additional tools. For example, the substrate may be a sheet of paper, which is available to users. Furthermore, the camera of the mobile device may be used for capturing the digital image of the fecal sample. The tools used for getting the fecal sample (e.g. scoop) or smearing the fecal sample on the substrate for obtaining a fecal streak (e.g. blade) may also be easily available to the users. In some examples, these tools may be provided to the users by means of a kit of parts.

Optionally, the method is used for monitoring temporal microbiome variations. The machine learning model, for instance a deep neural network model, may be used to track changes in fecal microbiota in relation to fecal images over time.

According to an aspect, the disclosure provides for a method for training a machine learning model for analyzing intestinal microflora of a subject with digital images of fecal samples, the method including: a) receiving a data set comprising a plurality of digital images of fecal samples; b) receiving measurement data representative of abundances of the predetermined microbial enterotype, species or genera in the fecal sample in each digital image of the plurality of digital images, wherein the predetermined microbial genera include one or more microbial enterotypes or microbial abundance profiles including prevotella and bacteroides; and c) training the machine learning data processing model based on the date received in step b) and the digital images received in step a) for enabling, after completion of the training period, the step of automatically associating abundances of predetermined microbial enterotype, species, or genera with digital images of fecal samples.

Determining the composition of human gut microbiota (e.g. designated enterotypes) may be a cumbersome task, requiring a lot of resources. For instance, DNA/RNA sequencing may be performed. By employing an image of the fecal sample, for instance a fecal streak, the training efficiency of the machine learning model may be improved. As a result, the involved costs for training the machine learning model may be lower. The disclosure provides for a cost-effective solution for monitoring and/or analyzing the intestinal microflora of a subject.

Optionally, the measurement data includes any sequencing data, for example being such as a16S sequencing data, corresponding to the fecal sample in the digital image.

Optionally, the measurement data accompanying each digital image is normalized to a predetermined total abundance of the predetermined microbial genera.

According to an aspect, the disclosure provides for a system for analyzing intestinal microflora of a subject according to claim 7.

Advantageously, the system can provide for a rapid and simple mechanism for identifying microbial phenotypes which are important in long-term metabolic health based on gut microbiota.

According to an aspect, the disclosure provides for a computer program product configured for performing, when run on a computer, the steps of the method according to the disclosure.

According to an aspect, the disclosure provides for a trained machine learning model, the trained machine learning model being configured for use in a method for analyzing intestinal microflora of a subject, wherein the trained machine learning model is configured to receive an image of a fecal sample of a subject as input and provide a classification as output, the classification being indicative of one or more properties of the intestinal microflora of the subject.

Optionally, the computer program is a mobile app which is executable on a mobile device, such as a mobile phone or a tablet. In some examples, the computer program is executable on a personal computer, such as a Windows, Macintosh (Apple), Linux, and/or Unix machine.

According to an aspect, the disclosure provides for a processor implemented method for determining an intestinal microbial composition of a subject, comprising: receiving, by one or more processors, a digital image of a sample of feces of the subject; and determining, by the one or more processors, the intestinal microbial composition of the subject on the basis of inferred patterns between the image and its corresponding microbial profile obtained for example by sequencings procedure. Various high sequencing procedures may be used, for instance high throughput sequencing procedures. Optionally, the pattern determination is performed using a machine learning data processing model that has been trained to associate abundances of the predetermined microbial species, genera and other phylogenetic information with digital images of fecal samples.

A deep learning neural network can be seen as a representation-learning method with a plurality of levels of representation, which can be obtained by composing simple but non-linear modules that each transform the representation at one level, starting with the raw input, into a representation at a higher, slightly more abstract level. The deep neural network may identify patterns which are difficult to see using conventional or classical methods. Hence, instead of writing custom code specific to a problem of identifying visual patterns in the images of the fecal sample of the subject, the network can be trained to be able to handle different and/or changing images of the feces sample e.g. using a classification algorithm. Training data may be fed to the deep neural network such that it can determine a classification logic for efficiently analyzing intestinal microflora of the subject.

It will be appreciated that any of the aspects, features and options described in view of the methods apply equally to the systems and the described devices, computer program products. It will also be clear that any one or more of the above aspects, features and options can be combined.

### BRIEF DESCRIPTION OF THE DRAWING

The disclosure will further be elucidated on the basis of exemplary embodiments which are represented in a drawing. The exemplary embodiments are given by way of non-limitative illustration. It is noted that the figures are only schematic representations of embodiments of the disclosure that are given by way of non-limiting example.

In the drawing:
Fig. 1 shows a schematic diagram of a system;
Fig. 2 shows a schematic diagram of a system;
Fig. 3 shows a schematic diagram of a substrate with a fecal streak applied thereon;
Fig. 4 shows a schematic diagram of a substrate;
Fig. 5a, 5b show an image of a fecal streak on a substrate; and
Fig. 6 shows a schematic diagram of a method.

### DETAILED DESCRIPTION

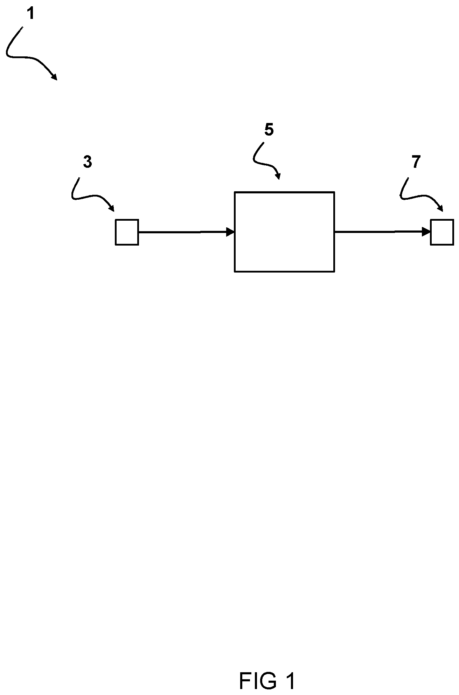

Fig. 1 shows a schematic diagram of a system 1 for analyzing intestinal microflora of a subject. The system 1 comprises one or more processors for receiving a digital image 3 and/or one or more features extracted from the digital image 3, which is provided as input to trained machine learning model 5, wherein the trained machine learning model 5 is configured to output a classification 7 based on the digital image 3 and/or one or more features extracted from the digital image 3. The one or more processors are configured to determine, data indicative of one or more properties (e.g. abundance levels) of the intestinal microflora of the subject based on the output image classification 7.

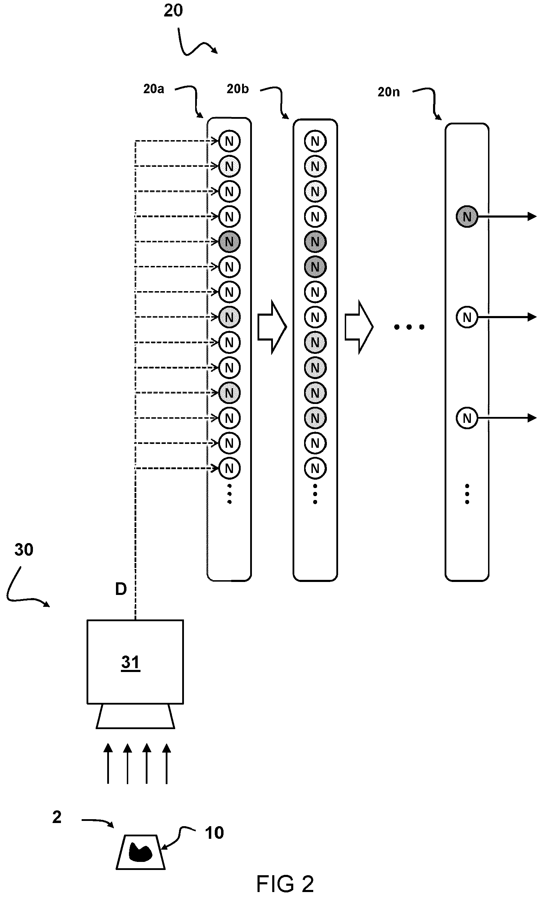

Fig. 2 shows a schematic diagram of an embodiment of a deep neural network 20 comprising a plurality of neurons "N". The neurons "N" are distributed over multiple layers 20a-n to form a deep neural network. Typically, such layers include an input layer 20a, an output layer 20n, and one or more one or more hidden layers 20b-(n-1) therebetween. The trained neural network can be configured to calculate output values Ox, Oy, Oz of neurons in the output layer 20n which may be based on a series of received input values based on the image of the sample of feces 2 of the subject. In this example, the sample of feces 2 is placed on a surface 10. The image may for instance be taken by means of a sensor unit 30. The sensor unit 30 can be a digital visual camera or the like. The camera may be a stand-alone camera, however, it is also possible to use camera of a mobile device, such as for instance a mobile phone, a tablet, or the like. In some examples, the image is taken in normal lighting conditions by placing the camera 31 substantially transverse with respect to the surface (e.g. substrate) on which the sample of feces 2 is placed. However, advantageously, in some embodiments, a transformation is performed on the captured image for projecting the image such as to compensate for visual effects due to the angle between the camera and the surface on which the sample of faces 2 is placed. This step can significantly improve the results when mobile devices are used for taking the images. Often, in such cases the relative position between the camera and the surface/substrate on which the feces is placed is sub-optimal.

In the shown example, the neural network 20 comprises an input layer 20a with neurons configured to receive input values corresponding to one or more images of the sample of the feces of the subject on a surface (e.g. substrate). For instance, the feces can be put on a substrate, such as for instance a piece of paper. In some examples, the sample of feces is smeared out on the substrate/surface such as to become a streak of feces. It will be appreciated that other sensory data or data derived therefrom can be used as input. In some embodiments, features extracted from the images (e.g. by performing image processing steps) are provided as input to the deep neural network.

In some embodiments, e.g. as shown, interconnections between the neurons "N" are formed exclusively across subsequent layers, e.g. between the input layer 20a and the first hidden layer 20b, between the first hidden layer 20b and the second hidden layer 20c (not shown), etc., up to the last hidden layer (index n-1, not shown) and the output layer 20n. Also other configurations of networks and connections may be envisaged.

A controller of the system may be configured to communicate with the sensor unit 30 comprising at least one sensor device, e.g. camera 31, for registering sensor data representing a sample of the feces of the subject. In some examples, the camera 31 is placed above the sample of feces and a top image is taken. The controller can be also configured to comprise or communicate with the neural network 20. For example, neural network 20 is configured (and/or programmed) to receive the sensor data D, and process the sensor data D to calculate the one or more output values Ox, Oy, Oz, etc. (e.g. classification values representing a respective classification of the sample of feces).

Many other types sensor devices may be envisaged. Also combinations of two or more sensors can be used as input to a neural network (e.g. deep neural network). The two or more sensors may be of the same type or different types. For example, the system may comprise a number of different cameras, e.g. combination of visual camera and infrared camera.

Based on the sensor data registered by the sensor unit 30, the neural network may classify the data. In some embodiments, the classifications may be predetermined, and the neural network may be trained to recognized the classifications (supervised learning). In other embodiments, the neural network may itself determine a set of classifications that may then be labelled (unsupervised learning). In some examples, the output of the neural network may calculate a probability of classifications indicative of or related to one or more properties of the intestinal microflora of the subject.

The classification values Ox, Oy, Oz may serve as input to a further system or module which is to advise certain actions, such as for example health monitoring, personalized diet proposals, etc. It may also be served as input to further system components (not shown) which may take action (e.g. alert a health professional) or present options based on the classification. The classification values Ox, Oy, Oz may also be simply output to a user interface reporting the event (not shown).

Aspects of the present disclosure may also relate to corresponding methods of training a neural network. In one embodiment, the method comprises classifying one or more properties of the intestinal microflora of the subject based on an image of the sample of feces of the subject. In some examples, a (non-transitory) computer-readable medium can be provided with software instructions that, when executed, causes performing the method as described herein, or forming a network or computer system as described herein. It will be appreciated that analyzing steps may also be performed on cloud systems or platforms (e.g. server).

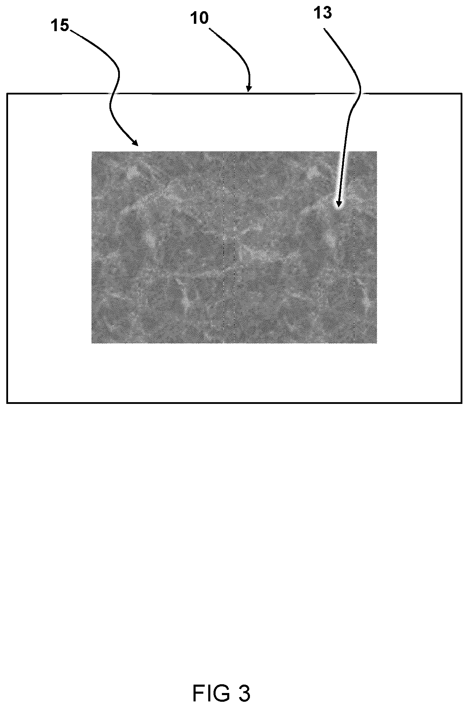

Fig. 3 shows a schematic diagram of a substrate 10 with a fecal streak 13 applied thereon. The fecal streak 13 has been applied on a portion or region 15 of the substrate 10. The substrate 10 with fecal streak 13 can be effectively used for analyzing and estimating intestinal microflora of a subject. A digital image of the substrate 10 with fecal streak 13 can be taken. The digital image and/or one or more features extracted from the digital image may be provided as input to a trained machine learning model which is configured to output a classification based on said input digital image and/or one or more features extracted from the digital image. Data indicative of one or more properties of the intestinal microflora of the subject can be determined based on the output image classification.

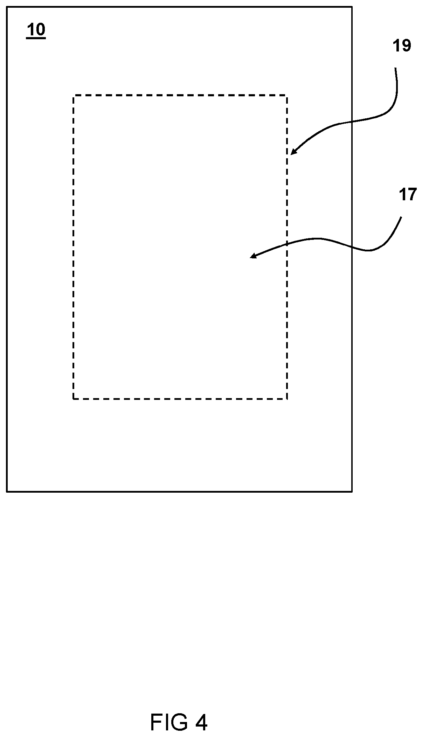

Fig. 4 shows a schematic diagram of a substrate 10. The substrate 10 has a region 17 which is visually indicated by marking lines 19 (dashed line) in which the fecal streak is to be provided. The substrate 10 may be a sheet of paper (e.g. standard A4 format), allowing the user to easily produce it (e.g. by printing a digital document such as a PDF document, Word Document, or the like). This is particularly advantageous when fecal samples are frequently monitored. The user can perform the analysis by means of a mobile device with an app. The app can be configured to receive the image of the sample of feces and upload it to a server or the cloud for analysis by means of the trained machine learning model. One or more results from the analysis can be fed back to the user. This also enables to track the evolution of the state of intestinal microflora of the subject over time (e.g. periodically sampling, such as for instance with time intervals of days, weeks or months).

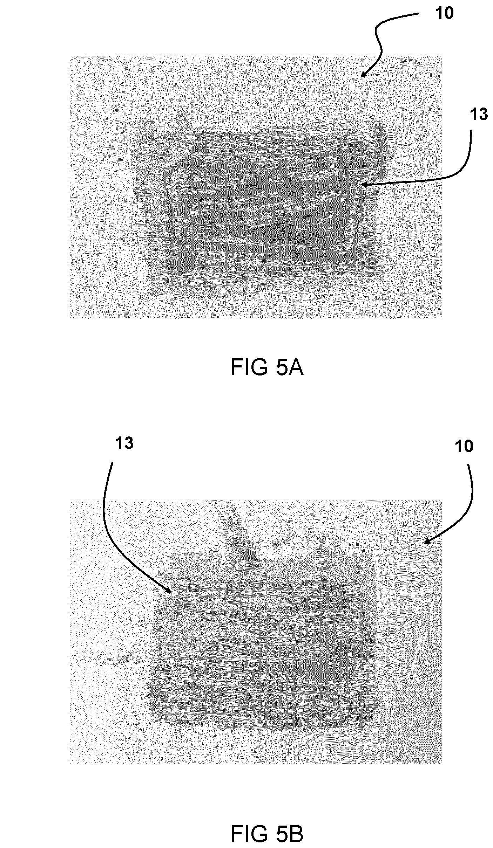

Fig. 5a, 5b show an image of a fecal streak on a substrate 10. Two examples of fecal streak pictures are shown. Fig. 5a shows an image of a fecal streak 13 on a substrate 10 which is prevotella rich, and fig. 5b shows an image of a fecal streak 13 on a substrate 10 which is bacteroides rich. Temporal microbiome variations may be associated with health status. In some examples, deep neural network models are used to explore changes in fecal microbiota in relation to fecal images over time. The picture of fecal streak shown in fig. 5a can be analyzed for composition and structure and linked to gutmicrobiota composition. Experiments were carried out with fecal samples comparing fecal streak with 16S sequencing. The trained machine learning model provided a good predictive value (AUC above 0.82) of fecal imaging with gutmicrobiota composition. In these exemplary experiments the deep learning image models are able identify patterns and association with abundances of gutmicrobial groups such as Prevotella/Bacteroides which are not detectable by the naked eye. Beyond just colour differences, the subtle features such as compositional changes, smaller particles of faces spread on the substrate 10, consistency, etc., all contribute to good classification score of the computational machine learning model. In this example, the substrate 10 was a A4 paper. Advantageously, the analysis is stable with respect to various noisy image background options and light conditions.

The machine learning model can be trained on 16s sequencing data and digital images of the fecal streaks (example above) samples, such as to identify microbial phenotypes such as fecal structure as well as presence and relative abundance of microbial species, or ratios between certain species in the sample. Advantageously, the machine learning model is used for visual evaluations of stool samples.

In an example, a dataset of fecal images is collected and subsequently used for training an image detection machine learning model. The dataset may contain images of fecal samples spread out on substrate, e.g. on a standard white A4 paper. The images may be accompanied by 16S sequencing data. The machine learning model can identify visual properties of the fecal samples which are linked to a subject's microbiome enterotype and/or subject's health. Such information can be subsequently used to establish nutritional guidelines based on images of the feces only. Advantageously, a highly efficient and effective system can be obtained in this way. In some examples, the training is performed with focus on the genus-level Prevotella/Bacteroides ratio.

In some examples, during training the images are augmented , i.e., transformed using random transformations, e.g. cropping, rotations, translations, color changes, or the like. The advantage of this is the models are able to learn more general properties of the images, instead of small subtle differences in form of noise. It improves the model's ability to generalize, or in other words be able to perform well on unseen data.

In some examples, the 16S sequencing data was preprocessed. The 16S sequencing data may be normalized to a point where each sample has equal read count. In some examples, all ASV's with less than 0.1 * N non-zero read counts were dropped, where N is the sample size. In some examples, all ASV's with an average read count of less than 3 were dropped. However, other values may also be used for these exemplary optional steps.

In some examples, as biological target, or phenotype, the Prevotella/Bacteroides ratio on genus level was selected. Advantageously, a high Prevotella/Bacteroides ratio is correlated with greater metabolic health. Low ratios may indicate a need in a higher intake of fibre rich food and plant-based protein, which is beneficial for the health of the subject.

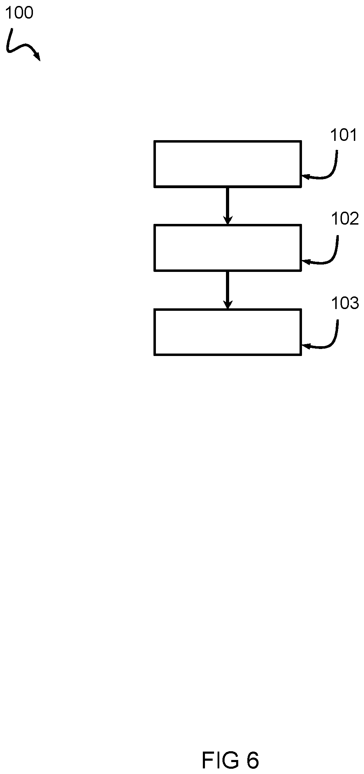

Fig. 6 shows a schematic diagram of a method 100 for analyzing intestinal microflora of a subject. In a first step 101, a digital image of a sample of feces of the subject is received by one or more processors. In a second step, the digital image and/or one or more features extracted from the digital image is provided as input to a trained machine learning model which is configured to output a classification based on said input digital image and/or one or more features extracted from the digital image. In a third step 103, data indicative of one or more properties of the intestinal microflora of the subject is determined based on the output image classification.

In some examples, a mobile device app is used, wherein the mobile device app is configured to allow uploading of a digital image of the sample of feces, preferably spread out on white paper. The images can be analyzed in real-time by the system according to the disclosure (e.g. cloud). In some examples, the users may automatically receive nutritional guidelines and a reasonable estimate of the overall health status of their gut microbiome based on the visual analysis performed by means of the trained machine learning model. However, it is also envisaged that the method is carried out by means of a website or software executed on a computer. Various implementations are possible.

It will be appreciated that various machine learning models may be used. In some examples, a deep learning model is employed. Deep learning allows computational models that are composed of multiple processing layers to learn representations of data with multiple levels of abstraction. In this way, visual pattern recognition may be significantly improved. Deep learning discovers intricate structure in large data sets by using the backpropagation algorithm to indicate how a machine should change its internal parameters that are used to compute the representation in each layer from the representation in the previous layer. Various deep learning networks and architectures may be employed.

It will be appreciated that the subject can be a human or animal (e.g. cattle). For both humans and animals an improved personalized diet can be more easily obtained, taking into account the condition of the intestinal microbiota of the subject. The individual health condition of the subjects can thus be significantly improved in an easy way by employing the method according to the disclosure.

It will be appreciated that the term microbiome may be understood as microorganisms found in the intestines of the subject. The term microbiome encompasses both the microbiota (community of microorganisms) and the theatre of activity (structural elements, and the surrounding environmental conditions).

It will be appreciated that in some examples in the description microbial genera is described. This may represent high level microbial information. However, additionally or alternatively, the method can also be employed with microbial species abundances.

In some examples, the digital image is directly provided as input to the trained machine learning model. Optionally, the digital image is first preprocessed prior to providing it as input to the trained machine learning model, for example involving segmentation (selection of certain portions of data in the digital image overlapping with one or more identified features in the image; filtering; etc). In some example, the pixels of the digital image are provided to the nodes of the input layer of the trained deep learning model.

It will be appreciated that the method may include computer implemented steps. All above mentioned steps can be computer implemented steps. Embodiments may comprise computer apparatus, wherein processes performed in computer apparatus. The disclosure also extends to computer programs, particularly computer programs on or in a carrier, adapted for putting the disclosure into practice. The program may be in the form of source or object code or in any other form suitable for use in the implementation of the processes according to the disclosure. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

It will be further understood that when a particular step of a method is referred to as subsequent to another step, it can directly follow said other step or one or more intermediate steps may be carried out before carrying out the particular step, unless specified otherwise. Likewise it will be understood that when a connection between components such as neurons of the neural network is described, this connection may be established directly or through intermediate components such as other neurons or logical operations, unless specified otherwise or excluded by the context.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

The graphics and/or image/video processing techniques may be implemented in various hardware architectures. Graphics functionality may be integrated within a chipset. Alternatively, a discrete graphics processor may be used. For example, processing of images (still or video) may be performed by a graphics subsystem such as a graphics processing unit (GPU) or a visual processing unit (VPU). As still another embodiment, the graphics or image/video processing functions may be implemented by a general purpose processor, including e.g. a multi-core processor. In a further embodiment, the functions may be implemented in a consumer electronics device. Embodiments, using a combination of different hardware architectures are possible.

In various embodiments, the controller can communicate using wireless systems, wired systems, or a combination of both. When implemented as a wired system, the system may include components and interfaces suitable for communicating or wired communications media, such as input/output (I/O) adapters, physical connectors to connect the I/O adapter with a corresponding wired communications medium. When implemented as a wireless system, the system may include components and interfaces suitable for communicating over a wireless shared media, such as one or more antennas, transmitters, receivers, transceivers, amplifiers, filters, control logic, and so forth. An example of wireless shared media may include portions of a wireless spectrum, such as the RF spectrum and so forth. A wireless communication device may be included in order to transmit and receive signals using various suitable wireless communications techniques. Such techniques may involve communications across one or more wireless networks. Exemplary wireless networks include, but are not limited to, cellular networks, wireless local area networks (WLANs, cfr. WiFi), wireless personal area networks (WPANs), wireless metropolitan area network (WMANs), satellite networks, et cetera. In communicating across such networks, the transmitter may operate in accordance with one or more applicable standards in any version.

Herein, the disclosure is described with reference to specific examples of embodiments of the disclosure. For the purpose of clarity and a concise description features are described herein as part of the same or separate embodiments, however, alternative embodiments having combinations of all or some of the features described in these separate embodiments are also envisaged and understood to fall within the framework of the disclosure as outlined by the claims. The specifications, figures and examples are, accordingly, to be regarded in an illustrative sense rather than in a restrictive sense. The disclosure is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

## Claims

1. A method (100) for analyzing intestinal microflora of a subject, comprising:
Receiving (101), by one or more processors, a digital image (3) of a sample of feces (2) of the subject;
providing the digital image and/or one or more features extracted from the digital image as input to a trained machine learning model (5) which is configured to output a classification (7) associated to abundance levels of the intestinal microflora based on said input digital image and/or one or more features extracted from the digital image; and
determining (103), by the one or more processors, data indicative of abundance levels of the intestinal microflora of the subject based on the output image classification, wherein the digital image is an image of a fecal streak (13) on a substrate (10),
**characterized in that** the fecal streak is obtained by smearing a layer of feces on the substrate and the substrate is a sheet of paper;
the image classification is associated to abundances of predetermined microbial species, genera and other phylogenetic information, wherein the predetermined microbial genera include one or more microbial enterotypes or microbial abundance profiles including prevotella and bacteroides;
and that the method further includes determining, based on the output image classification, relative abundances of the one or more predetermined microbial enterotypes, species or genera, including a ratio of the abundance of prevotella genera and the abundance of bacteroides genera.

2. The method of claim 1, wherein the substrate (10) and/or an area covered by the fecal streak (13) have dimensions falling within a predetermined range.

3. A method of determining an indication of a subject's health based on data indicative of one or more properties of the intestinal microflora of the subject determined according to any of the preceding claims 1-2

4. The method according to claim 3, wherein the digital image (3) of the sample of feces (2) of the subject is taken by means of a mobile device and uploaded to a server, wherein the server is configured to carry out the method according to any one of the claims 1-2.

5. A method for training a machine learning model (5) for analyzing intestinal microflora of a subject with digital images (3) of fecal samples, the method including:
a) receiving a data set comprising a plurality of digital images (3) of fecal samples on paper substrates;
b) receiving data representative of abundances of the predetermined microbial enterotype, species or genera in the fecal sample in each digital image of the plurality of digital images, wherein the predetermined microbial genera include one or more microbial enterotypes or microbial abundance profiles including prevotella and bacteroides; and
c) training the machine learning data processing model (5) based on the data received in step b) and the digital images (3) received in step a) for enabling, after completion of the training period, the step of automatically associating abundances of predetermined microbial enterotype, species, or genera with digital images of fecal samples, wherein the trained machine learning model is used for analyzing intestinal microflora of a subject according to the method of any one of claims 1-4.

6. A system (1) for analyzing intestinal microflora of a subject, the system comprising:
one or more processors for receiving a digital image (3) of a sample of feces (2) of the subject; and
a memory storing a trained machine learning model (5), wherein the trained machine learning model is configured to output a classification (7) associated to abundance levels of the intestinal microflora based on the digital image and/or one or more features extracted from the digital image (3) provided as input; and
wherein the one or more processors are configured to determine, data indicative of abundance levels of the intestinal microflora of the subject based on the output image classification;
**characterized in that**
the trained machine learning model (5) is configured to output a classification associated to abundances of predetermined microbial species, genera and other phylogenetic information, wherein the predetermined microbial genera include one or more microbial enterotypes or microbial abundance profiles including prevotella and bacteroides
and that the one or more processors are further configured to determine, based on the output image classification, relative abundances of the one or more predetermined microbial enterotypes, species or genera, including a ratio of the abundance of prevotella genera and the abundance of bacteroides genera.

## Patentansprüche

1. Verfahren (100) zur Analyse der Darmmikroflora eines Subjekts, umfassend:
Empfangen (101) eines digitalen Bildes (3) einer Probe von Fäzes (2) des Subjekts durch einen oder mehrere Prozessoren;
Bereitstellen des digitalen Bildes und/oder eines oder mehrerer aus dem digitalen Bild extrahierter Merkmale als Eingabe für ein trainiertes maschinelles Lernmodell (5), das dazu konfiguriert ist, auf der Grundlage des genannten Eingabe-Digitalbildes und/oder eines oder mehrerer aus dem digitalen Bild extrahierter Merkmale eine Klassifikation (7) auszugeben, die mit Häufigkeitsniveaus der Darmmikroflora assoziiert ist; und
Bestimmen (103) von Daten, die auf Häufigkeitsniveaus der Darmmikroflora des Subjekts hinweisen, durch den einen oder die mehreren Prozessoren auf der Grundlage der ausgegebenen Bildklassifikation, wobei das digitale Bild ein Bild eines Fäzesstrichs (13) auf einem Substrat (10) ist,
**dadurch gekennzeichnet, dass** der Fäzesstrich durch Verschmieren einer Schicht von Fäzes auf dem Substrat erhalten wird und das Substrat ein Blatt Papier ist;
die Bildklassifikation mit Häufigkeiten vorbestimmter mikrobieller Spezies, Gattungen und anderer phylogenetischer Informationen assoziiert ist, wobei die vorbestimmten mikrobiellen Gattungen einen oder mehrere mikrobielle Enterotypen oder mikrobielle Häufigkeitsprofile einschließlich Prevotella und Bacteroides umfassen;
und dass das Verfahren ferner das Bestimmen relativer Häufigkeiten der einen oder mehreren vorbestimmten mikrobiellen Enterotypen, Spezies oder Gattungen auf der Grundlage der ausgegebenen Bildklassifikation umfasst, einschließlich eines Verhältnisses der Häufigkeit von Prevotella-Gattungen zu der Häufigkeit von Bacteroides-Gattungen.

2. Verfahren nach Anspruch 1, wobei das Substrat (10) und/oder ein von dem Fäzesstrich (13) bedeckter Bereich Abmessungen aufweisen, die innerhalb eines vorbestimmten Bereichs liegen.

3. Verfahren zum Bestimmen einer Angabe der Gesundheit eines Subjekts auf der Grundlage von Daten, die auf eine oder mehrere Eigenschaften der Darmmikroflora des Subjekts hinweisen und die gemäß einem der vorhergehenden Ansprüche bestimmt wurden.

4. Verfahren nach Anspruch 3, wobei das digitale Bild (3) der Probe von Fäzes (2) des Subjekts mittels eines mobilen Geräts aufgenommen und auf einen Server hochgeladen wird, wobei der Server dazu konfiguriert ist, das Verfahren nach einem der Ansprüche 1-2 durchzuführen.

5. Verfahren zum Trainieren eines maschinellen Lernmodells (5) zur Analyse der Darmmikroflora eines Subjekts mit digitalen Bildern (3) von Fäzesproben, wobei das Verfahren Folgendes umfasst:
a) Empfangen eines Datensatzes, der eine Vielzahl digitaler Bilder (3) von Fäzesproben auf Papiersubstraten umfasst;
b) Empfangen von Daten, die für Häufigkeiten des vorbestimmten mikrobiellen Enterotyps, der Spezies oder der Gattungen in der Fäzesprobe in jedem digitalen Bild der Vielzahl digitaler Bilder repräsentativ sind, wobei die vorbestimmten mikrobiellen Gattungen einen oder mehrere mikrobielle Enterotypen oder mikrobielle Häufigkeitsprofile einschließlich Prevotella und Bacteroides umfassen; und
c) Trainieren des maschinellen Lern-Datenverarbeitungsmodells (5) auf der Grundlage der in Schritt b) empfangenen Daten und der in Schritt a) empfangenen digitalen Bilder (3), um nach Abschluss des Trainingszeitraums den Schritt des automatischen Assoziierens von Häufigkeiten vorbestimmter mikrobieller Enterotypen, Spezies oder Gattungen mit digitalen Bildern von Fäzesproben zu ermöglichen, wobei das trainierte maschinelle Lernmodell zur Analyse der Darmmikroflora eines Subjekts gemäß dem Verfahren nach einem der Ansprüche 1-4 verwendet wird.

6. System (1) zur Analyse der Darmmikroflora eines Subjekts, wobei das System umfasst:
einen oder mehrere Prozessoren zum Empfangen eines digitalen Bildes (3) einer Probe von Fäzes (2) des Subjekts; und
einen Speicher, der ein trainiertes maschinelles Lernmodell (5) speichert, wobei das trainierte maschinelle Lernmodell dazu konfiguriert ist, eine Klassifikation (7) auszugeben, die mit Häufigkeitsniveaus der Darmmikroflora auf der Grundlage des als Eingabe bereitgestellten digitalen Bildes und/oder eines oder mehrerer aus dem digitalen Bild (3) extrahierter Merkmale assoziiert ist; und
wobei der eine oder die mehreren Prozessoren dazu konfiguriert sind, Daten zu bestimmen, die auf Häufigkeitsniveaus der Darmmikroflora des Subjekts auf der Grundlage der ausgegebenen Bildklassifikation hinweisen;
**dadurch gekennzeichnet, dass**
das trainierte maschinelle Lernmodell (5) dazu konfiguriert ist, eine Klassifikation auszugeben, die mit Häufigkeiten vorbestimmter mikrobieller Spezies, Gattungen und anderer phylogenetischer Informationen assoziiert ist, wobei die vorbestimmten mikrobiellen Gattungen einen oder mehrere mikrobielle Enterotypen oder mikrobielle Häufigkeitsprofile einschließlich Prevotella und Bacteroides umfassen,
und dass der eine oder die mehreren Prozessoren ferner dazu konfiguriert sind, auf der Grundlage der ausgegebenen Bildklassifikation relative Häufigkeiten der einen oder mehreren vorbestimmten mikrobiellen Enterotypen, Spezies oder Gattungen zu bestimmen, einschließlich eines Verhältnisses der Häufigkeit von Prevotella-Gattungen zu der Häufigkeit von Bacteroides-Gattungen.

## Revendications

1. Procédé (100) d'analyse de la microflore intestinale d'un sujet, comprenant les étapes suivantes :
recevoir (101) par un ou plusieurs processeurs une image numérique (3) d'un échantillon de fèces (2) du sujet ;
fournir l'image numérique et/ou une ou plusieurs caractéristiques extraites de l'image numérique comme entrée dans un modèle d'apprentissage machine (5) entraîné qui est configuré pour délivrer une classification (7) associée à des niveaux d'abondance de la microflore intestinale sur la base de ladite image numérique d'entrée et/ou desdites une ou plusieurs caractéristiques extraites de l'image numérique ; et
déterminer (103) par les un ou plusieurs processeurs des données indicatives des niveaux d'abondance de la microflore intestinale du sujet sur la base de la classification d'image de sortie, dans lequel l'image numérique est une image d'une traînée fécale (13) sur un substrat (10),
**caractérisé en ce que** la traînée fécale est obtenue en étalant une couche de fèces sur le substrat, et le substrat est une feuille de papier ;
la classification d'image est associée à l'abondance d'espèces et de genres microbiens prédéterminés et à d'autres informations phylogénétiques, dans lequel les genres microbiens prédéterminés comportent un ou plusieurs entérotypes microbiens ou profils d'abondance microbienne comportant la Prevotella et les Bacteroïdes ;
et **en ce que** le procédé comporte en outre, sur la base de la classification d'image de sortie, le fait de déterminer l'abondance relative des un ou plusieurs entérotypes, des espèces ou des genres microbiens prédéterminés comportant un rapport entre l'abondance du genre Prevotella et l'abondance du genre Bacteroïdes.

2. Procédé selon la revendication 1, dans lequel les dimensions du substrat (10) et/ou d'une zone recouverte par la traînée fécale (13) s'inscrivent dans une plage prédéterminée.

3. Procédé destiné à déterminer une indication de la santé d'un sujet sur la base de données indicatives d'une ou plusieurs propriétés de la microflore intestinale du sujet déterminées selon l'une des revendications 1 et 2 précédentes.

4. Procédé selon la revendication 3, dans lequel l'image numérique (3) de l'échantillon de fèces (2) du sujet est prise au moyen d'un dispositif mobile et téléchargée sur un serveur, dans lequel le serveur est configuré pour mettre en œuvre le procédé selon l'une des revendications 1 et 2.

5. Procédé d'entraînement d'un modèle d'apprentissage machine (5) destiné à analyser la microflore intestinale d'un sujet à l'aide d'images numériques (3) d'échantillons fécaux, le procédé comportant les étapes suivantes :
a) recevoir un ensemble de données comprenant une pluralité d'images numériques (3) d'échantillons fécaux sur des substrats en papier ;
b) recevoir des données représentatives de l'abondance des entérotypes, des espèces ou des genres microbiens prédéterminés dans l'échantillon fécal dans chaque image numérique de la pluralité d'images numériques, dans lequel les genres microbiens prédéterminés comportent un ou plusieurs entérotypes microbiens ou profils d'abondance microbienne comportant la *Prevotella* et les *Bacteroïdes* ; et
c) entraîner le modèle d'apprentissage machine (5) de traitement de données sur la base des données reçues à l'étape b) et des images numériques (3) reçues à l'étape a) afin de permettre, à l'issue de la période d'entraînement, d'effectuer l'étape d'association automatique de l'abondance des entérotypes, des espèces ou des genres microbiens prédéterminés à des images numériques d'échantillons fécaux, dans lequel le modèle d'apprentissage machine entraîné est utilisé pour analyser la microflore intestinale d'un sujet selon le procédé de l'une des revendications 1 à 4.

6. Système (1) d'analyse de la microflore intestinale d'un sujet, le système comprenant :
un ou plusieurs processeurs pour recevoir une image numérique (3) d'un échantillon de fèces (2) du sujet ; et
une mémoire stockant un modèle d'apprentissage machine (5) entraîné, dans lequel le modèle d'apprentissage machine entraîné est configuré pour délivrer une classification (7) associée à des niveaux d'abondance de la microflore intestinale sur la base de l'image numérique et/ou des une ou plusieurs caractéristiques extraites de l'image numérique (3) fournie comme entrée ; et
dans lequel les un ou plusieurs processeurs sont configurés pour déterminer des données indicatives de niveaux d'abondance de la microflore intestinale du sujet sur la base de la classification d'image de sortie ;
**caractérisé en ce que**
le modèle d'apprentissage machine (5) entraîné est configuré pour délivrer une classification associée à l'abondance d'espèces et de genres microbiens prédéterminés et à d'autres informations phylogénétiques, dans lequel les genres microbiens prédéterminés comportent un ou plusieurs entérotypes microbiens ou profils d'abondance microbienne comportant la *Prevotella* et les *Bacteroïdes* ;
et **en ce que** les un ou plusieurs processeurs sont en outre configurés pour, sur la base de la classification d'image de sortie, déterminer l'abondance relative des un ou plusieurs entérotypes, des espèces ou des genres microbiens prédéterminés comportant un rapport entre l'abondance du genre Prevotella et l'abondance du genre Bacteroïdes.
